# EUROPEAN PATENT SPECIFICATION

(11) **EP 1 175 901 B1**
(45) Date of publication and mention of the grant of the patent: **31.05.2006**
(21) Application number: 00915522.7
(22) Date of filing: 13.04.2000
(51) Int. Cl.: A61K 31/202

(54) **PEROXISOME ACTIVATOR-RESPONSIVE RECEPTOR AGONISTS**
AGONISTEN DES PEROXISOM AKTIVATOR-RESPONSIVEN REZEPTORS
AGONISTES DU RECEPTEUR SENSIBLE A L'ACTIVATEUR DU TYPE PEROXYSOME

(30) Priority: 15.04.1999 JP 10858899
(43) Date of publication of application: 30.01.2002
(73) Proprietor: KANEKA CORPORATION, Osaka-shi, Osaka 530-0005 (JP)
(72) Inventor: KAWADA, Teruo, Uji-shi, Kyoto 611-0031 (JP); MIYASHITA, Kazuo, Hakodate-shi, Hokkaido 041-0835 (JP); SHIRAISHI, Tadayoshi, Takasago-shi, Hyogo 676-0025 (JP); ABE, Masayuki, Takasago-shi, Hyogo 676-0801 (JP); KATO, Masakazu, Akashi-shi, Hyogo 674-0094 (JP); OFUJI, Takehiko, Kobe-shi, Hyogo 655-0039 (JP)
(74) Representative: Vossius & Partner
(86) International application number: PCT/JP2000/002429
(87) International publication number: WO 2000/062772

(56) References cited:
- EP-A- 0 606 012
- JP-A- 9 023 817
- JP-A- 63 222 659
- US-A- 4 851 437
- PALMER C N A ET AL: "cis-Parinaric acid is a ligand for the human peroxisome proliferator activated receptor gamma: development of a novel spectrophotometric assay for the discovery of PPARgamma ligands" FEBS LETTERS, ELSEVIER SCIENCE PUBLISHERS, AMSTERDAM, NL, vol. 431, no. 3, 24 July 1998 (1998-07-24), pages 476-480, XP004258182 ISSN: 0014-5793
- DHAR P ET AL: "NUTRITIONAL CHARACTERISTICS OF OIL CONTAINING CONJUGATED OCTADECATRIENOIC FATTY ACID" ANNALS OF NUTRITION AND METABOLISM, KARGER, CH, vol. 42, no. 5, September 1998 (1998-09), pages 290-296, XP008017572 ISSN: 0373-0101
- DHAR P ET AL: "DIETARY EFFECTS OF CONJUGATED OCTADECATRIENOIC FATTY ACID (9 CIS, 11 TRANS, 13 TRANS) LEVELS ON BLOOD LIPIDS AND NONENZYMATIC IN VITRO LIPID PEROXIDATION IN RATS" LIPIDS, CHAMPAIGN, IL, US, vol. 34, no. 2, February 1999 (1999-02), pages 109-114, XP000973904 ISSN: 0024-4201
- CORNELIUS A.S. ET AL.: 'Cytotoxic effect of cis-parinaric acid in cultured malignant cells' CANCER RESEARCH, vol. 51, 1991, pages 6025 - 6030, XP002929575
- LIN Q. ET AL.: 'Ligand selectivity of the peroxisome proliferator-activated receptor alpha' BIOCHEMISTRY, vol. 38, 1999, pages 185 - 190, XP002929576

## Description

### TECHNICAL FIELD

The present invention relates to an agonist having an activity of promoting the binding of a peroxisome proliferator-activated receptor (hereinafter abbreviated to 'PPAR') to a target gene sequence and promoting gene expression downstream thereof (the agonist is hereinafter referred to as a 'PPAR agonist', and the activity as 'PPAR agonist activity'), and to the use of the PPAR agonist in reducing visceral fat, suppressing visceral fat accumulation, ameliorating lipid metabolism abnormalities, ameliorating glucose metabolism abnormalities, or in humans or animals, especially mammals.

### BACKGROUND ART

Due to an aging society, there is an increasing need for foods/beverages and medicines that are effective in preventing/treating lifestyle-related disorders of humans, representative examples of which are diabetes, atherosclerosis, hypertension, hyperlipidemia, fatty liver and cancer. Moreover, obesity and diabetes in pets is a problem nowadays, and there are calls for the development of feeds and veterinary medicines for preventing/treating these disorders. Due to recent advances in obesity research, it has become increasingly clear that obesity - and in particular visceral fat accumulation type obesity - is an important risk factor in lifestyle-related disorders (Takahashi et al., Program and Abstracts of the 19^{th} Japanese Obesity Conference, p64, 1998 (in Japanese)). It is thus hoped that it may well be possible to treat or ameliorate lifestyle-related disorders by ameliorating visceral fat accumulation type obesity.

Adipose tissue, which is the principal organ of visceral fat accumulation, was viewed merely as a place for storing excess fat in the past. However, due to recent advances in adipocyte research, it has become clear that adipose tissue is not merely a place to store the body's excess energy, but rather is also an organ that secretes various physiologically active substances and thus plays an active role in energy regulation. It is thought that PPARs, which are intranuclear receptors, play an important role in functional regulation in the adipose tissue. PPARs are so named because they are activated by compounds of various structures that have an action of proliferating peroxisomes, which are intracellar organelles (Issemann et al., Nature, 347, 645-650). c-DNA from various animals including humans and tissues has been cloned, and at present 3 subtypes, α, β and γ, of PPAR are known, with these being called PPARα, PPARβ (or sometimes PPARδ, FAAR or NUC1) and PPARγ respectively. PPARα appears mainly in hepatocytes, PPARβ is found in abundance in the heart, the lungs and the kidneys, and PPARγ is found in abundance in adipocytes. All of the subtypes of PPAR carry out their functions by forming a hetero-dimer with a retinoid X receptor (RXR), binding to a PPAR responsive sequence upstream of a target gene, and regulating transcription of the target gene (Lemberger et al., Ann. Rev. Cell Dev. Biol., 12, 335-363, 1996). Examples of known target genes of PPARs include the gene cluster for satiety factor leptin, lipoprotein lipase, which is involved in the uptake of fatty acids, fatty acid transporters, fatty acid binding proteins involved in the transport of fatty acids, malic enzymes, which are involved in fatty acid synthesis, phosphoenolpyruvate carboxykinase, acyl-CoA synthetase, which is involved in β oxidation, acyl-CoA oxidase, ketoacyl-CoA thiolase, and the like, and also the gene for uncoupling protein 1 (UCP-1), which is involved in thermogenesis in brown adipose tissue. All of these target gene products are proteins important in the control of lipid metabolism, glucose metabolism and energy metabolism in the body, and hence it is thought that PPARs are important proteins in the control of lipid synthesis, decomposition and metabolism, glucose metabolism, and energy metabolism in the body (Kawada, Igaku no Ayumi, 184, 519, 1998 (in Japanese); Kawada, Rinshoi, 23, 1300, 1997 (in Japanese); Shinra, Igaku no Ayumi, 184, 513, 1998 (in Japanese)). In particular, with regard to PPARγ, as a result of experiments carried out by Tontonz et al. into the expression of a PPARγ gene when introduced into fibroblasts, it has been shown that PPARγ is an important factor in the control of adipocyte differentiation (Cell, 79, 1147-1156, 1994). It is thus anticipated that substances showing PPAR agonist activity should enhance decomposition and metabolism of lipids, enhance energy metabolism, reduce body fat, in particular visceral fat, and ameliorate lipid metabolism abnormalities (Kawada, Igaku no Ayumi, 184, 519, 1998 (in Japanese); Kawada, Rinshoi, 23, 1300, 1997 (in Japanese); Shinra, Igaku no Ayumi, 184, 513, 1998 (in Japanese)).

Moreover, it is known that thiazolidines, which are PPARγ agonists, considerably ameliorate glucose metabolism abnormalities such as insulin resistance in type II diabetes patients. The mechanism thereof has not been sufficiently elucidated, but it is thought that PPARγ agonists promote differentiation of adipocytes, and as a result inhibit the production of TNFα, which is an insulin resistance inducing substance, promote glucose transporter (Glut4) expression in peripheral tissue, and reduce the amount of free fatty acids, and that as a result glucose uptake into cells is enhanced, and hyperglycemia is ameliorated (Kawada, Saishin Igaku, 53, 402, 1998 (in Japanese); Araki et al., Igaku no Ayumi, 188, 500, 1999 (in Japanese); J.M. Lehmann et al., J. Biol. Chem., 270, 12953, 1995; T. Fujiwara et al.; Diabetes, 37, 1549, 1988). Moreover, it has been reported that PPAR agonists stop cell proliferation and promote cell differentiation (S. Kitamura et al., Jpn. J. Cancer Res., 90, 75, 1999).

It is well known that visceral fat accumulation, lipid metabolism abnormalities and glucose metabolism abnormalities are important risk factors in lifestyle-related disorders such as hyperlipidemia, diabetes, hypertension, atherosclerosis, fatty liver and heart disease. Substances that reduce these risk factors are thus seen as being promising as food/beverage, medicinal and animal feed compositions exhibiting effects of preventing or ameliorating various lifestyle-related disorders (Mizukami et al., Saishin Igaku, 53, 402, 1998 (in Japanese)).

True PPAR agonists that fulfill a physiological function in the body are yet to be identified, but long-chain fatty acids, leukotriene B4, clofibrates and carbaprostacyclin are known as substances exhibiting PPARα agonist activity, long-chain fatty acids, carbaprostacyclin and bezafibrine acid as substances exhibiting PPARβ agonist activity, and thiazolidines (e.g. troglitazone), 15-deoxy-Δ^{12,14}-prostaglandin J₂, indomethacin and highly unsaturated fatty acids as substances exhibiting PPARγ agonist activity (Kawada, Saishin Igaku, 53, 402, 1998 (in Japanese); Kawada, Igaku no Ayumi, 184, 519, 1998 (in Japanese); S.A. Kliewer et al., Proc. Natl. Acad. Sci. USA, 94, 4318, 1997). Moreover, it has recently been reported that conjugated linoleic acid (hereinafter abbreviated to 'CLA') exhibits PPARα, β and γ agonist activity (K.L. Houseknecht, Biochem. Biophys. Res. Comm., 244, 678, 1998; S.Y. Moya-Camarena et al., Biochemica et Biophysica Acta, 1436, 331, 1999). As is clear from these reports, the ligand selectivity among the various PPAR subtypes is quite broad, and one of the characteristics of PPARs is that a substance that acts as an agonist for one particular subtype is often found to exhibit agonist activity toward the other subtypes.

Of substances that exhibit PPAR agonist activity, CLA and fish oils containing high concentrations of docosahexaenoic acid (hereinafter abbreviated to 'DHA') have also been reported to have dieting effects (K.L. Houseknecht, Biochem. Biophys. Res. Comm., 244, 678, 1998; Published Japanese Translation of PCT Application No. H10-508189; T. Kawada et al., J. Agr. Food Chem., 46, 1225, 1998). CLA is marketed as a health food under the brand names Tonalin (registered trademark) and *Kassei Rinooru* (registered trademark), and fish oils containing large amounts of DHA are also marketed as health food ingredients. However, CLA and high-DHA-containing fish oils do not have sufficient visceral fat reduction effects or visceral fat accumulation suppression effects, and fears remain regarding safety, for example it has been found that, when used in a dose sufficient to be effective, CLA has side-effects such as enlargement of the liver (D.B. West et al., Am. J. Physiol., 275, R667, 1998). Moreover, CLA and DHA both taste unpleasant, and hence are used as health foods in capsule form or the like, with many problems remaining with the use of CLA and DHA in general food/beverage, medicinal and animal feed compositions. Furthermore, CLA and DHA are both expensive, and hence there are limitations on usage in general food or animal feed compositions. There are thus calls for the development of food/beverage, medicinal and animal feed compositions that exhibit prominent visceral fat reduction effects and visceral fat accumulation prevention effects, and moreover are safe, strongly active, excellent in terms of taste and the like, and economical, as well as development of uses thereof.

Furthermore, clofibrates are used as drugs for ameliorating lipid metabolism abnormalities such as hyperlipidemia, and thiazolidines (e.g. TZD) are used as type II diabetes therapeutic drugs having insulin resistance amelioration effects, but there are still calls for safer and more effective novel compositions.

Moving on, it has been reported that cis-parinaric acid, which is a conjugated tetraenoic acid, binds to PPARγ protein, but PPARγ agonist activity has not been confirmed (C.N.A. Palmer and C.R. Wolf, FEBS Letters, 431, 476, 1998). Moreover, it is known that cis-parinaric acid only exists naturally in limited amounts from extremely limited sources such as balsam seed, and hence industrial use is problematic.

As described above, high-DHA-containing fish oils and CLA are conventionally known as oil compositions having a PPAR agonist activity, but either have poor visceral fat reduction effects or else suffer from problems in terms of taste, palatability, cost and the like when used in a food/beverage, and hence have not become widely used as food/beverage compositions. Moreover, clofibrates and thiazolidines are known as medicinal compositions, but do not have sufficient visceral fat reduction effects, with there being calls for compositions that have higher activity and are safer.

An object of the present invention is thus to provide methods of reducing visceral fat or suppressing visceral fat accumulation, ameliorating lipid metabolism abnormalities, ameliorating glucose metabolism abnormalities, and preventing or treating cancer, using a novel PPAR agonist having a high activity.

Parinaric acid has been reported to exert cytotoxic effects on malignant cells (Palmer and Wolf, FEBS letters, vol 431, 1998, p. 476-480, Cornelius et al, Cancer Res. vol 51, 1991, p. 6025-6030) and to be useful for the therapy of cancer or atherosclerosis (EP-A-0 606 012). The use of tung oil fatty acid compositions in the treatment of neoplastic diseases is disclosed in US-A-4,851,437. The nutritional characteristics and dietary effects of α-eleostearic acid are investigated in Dhar, et al, Lipids, vol. 34, 1999, p. 109 - 114 and Dhar et al Ann. Nutr. Metab. vol. 42, 1998, p. 290 - 296.

### DISCLOSURE OF THE INVENTION

The present inventors have carried out research over many years into food-related oils and fats, and in particular into the mechanism of differentiation of adipocytes, and substances that induce this differentiation and the functions thereof. During this research, the present inventors constructed a system for efficiently evaluating agonists for PPARγ, which is a representative subtype of PPAR, and evaluated the PPAR agonist activity of highly unsaturated fatty acids using this evaluation system. As a result, the inventors of the present invention discovered that conjugated trienoic acids and conjugated tetraenoic acids have a much higher PPAR agonist activity than conjugated dienoic acids such as CLA, and after carrying out further assiduous research based on this finding accomplished the present invention.

The peroxisome proliferator-activated receptor agonist of the present invention thus contains as an active ingredient punicic acid which is a conjugated unsaturated fatty acids having 18 carbon atoms and containing a conjugated trienoic structure (-CH=CH-CH=CH-CH=CH-) in the molecule thereof, and salts and ester derivatives thereof.

Moreover, the present invention may also be pomegranate seed oil composition containing the peroxisome proliferator-activated receptor agonist as described above.

The peroxisome proliferator-activated receptor agonist can be used as an active ingredient of a medicine, for reducing visceral fat or suppressing visceral fat accumulation, an agent for preventing or ameliorating lipid metabolism abnormalities.

Following is a more detailed description of the present invention. The PPAR agonist and the fat/oil composition containing the same of the present invention exhibit agonist activity toward PPARs, which are important factors at the top of a gene cascade involved in differentiation of adipocytes, synthesis, accumulation, metabolism and decomposition of lipids, control of glucose metabolism, and thermogenesis in the body. The PPAR agonist activity of the PPAR agonist and fat/oil composition of the present invention can be measured using an efficient method newly developed by the present inventors. In this method, a plasmid (pM-PPAR) that produces a chimeric protein of a DNA binding site of GAL4 (a DNA-binding transcription factor activation factor of yeast) and a ligand binding site of PPARγ which is expressed adipocyte specifically, or a plasmid (pM) in which PPARγ transmission has been removed from pM-PPAR, and a plasmid (4×UASg-luc) into which have been incorporated four GAL4 responsive sequences (USAg) upstream of a luciferase gene, which is a reporter gene, are cotransfected into cultured cells (CV-1) originating from the kidney of an African green monkey using lipofection (Kazuaki Yoshikawa, Gene Introduction/Expression Research Methods for Neurophysiology, Springer-Verlag Tokyo, 1997 (in Japanese)); the transformant thus obtained is cultured for a specific time period, and then by treating with an agonist candidate substance, the PPARγ-GAL4 chimeric protein is activated, binding to the USAg upstream of the 4×UASg-luc is enhanced, and as a result luciferase production is increased. The agonist activity can be evaluated by measuring the activity of the luciferase produced (Pica Gene Luminescence Kit instruction manual, Toyo B-Net. Co., Ltd.).

The visceral fat reduction effects or visceral fat accumulation suppression effects of the PPAR agonist and oil/fat composition of the present invention are evaluated by administering the candidate compound to a laboratory animal for a specific time period (e.g. 4 weeks), and then measuring the amount of visceral fat in the adipose tissue around the testes, the adipose tissue around the kidneys and the like.

The PPAR agonist used in the implementation of the present invention may exhibit agonist activity toward any of the PPAR subtypes since the PPAR ligand selectivity is extremely broad, but judging from the distinctive tissue distribution of each of the subtypes, it is particularly preferable for the PPAR agonist to exhibit agonist activity toward PPARγ. The PPAR agonist of the present invention contains, as an active ingredient exhibiting agonist activity, punicic acid, which is a conjugated unsaturated fatty acid having 18 carbon atoms and containing a conjugated trienoic structure (-CH=CH-CH=CH-CH=CH-) and salts and ester derivatives thereof.

Examples of conjugated unsaturated fatty acids exhibiting agonist activity include punicic acid (18:3, 9c, 11t, 13c), calendic acid (18:3, 8t, 10t, 12c), jarcaric acid (18:3, 8c, 10t, 12c), α-eleostearic acid (18:3, 9c, 11t, 13t), β-eleostearic acid (18:3, 9t, 11t, 13t), catalpic acid (18:3, 9t, 11t, 13c), kamlolenic acid (18OH, 9c, 11t, 13t), and other conjugated octadecatrienoic acids, as well as conjugated tetraenoic acids such as parinaric acid (18:4, 9c, 11t, 13t, 15c) and conjugated eicosatetraenoic acids. From the standpoint of safety and ease of acquisition, fatty acids having 18 carbon atoms and a conjugated trienoic structure are preferable, and from the standpoint of PPARγ agonist activity, punicic acid, which has a stereoisomerism of 9-cis, 11-trans, 13-cis, being particularly suitable.

The conjugated unsaturated fatty acid that makes up the active ingredient of the PPAR agonist of the present invention may be in the form of a salt or an ester derivative. A salt must be sitologically, nutritionally and pharmacologically acceptable, but otherwise there are no particular limitations; examples include metal salts such as a sodium salt and a potassium salt, an ammonium salt, salts with organic bases such as methylamine, ethylamine, diethylamine, triethylamine, pyrrolidine, piperidine, morpholine, hexamethyleneimine, aniline and pyridine, and salts with amino acids such as arginine, glutamic acid and ornithine. Moreover, an ester derivative must be sitologically, nutritionally and pharmacologically acceptable, but otherwise there are no particular limitations; an ethyl ester, a butyl ester, a propyl ester and a glycerol ester are preferable, with an ethyl ester and a glycerol ester being most preferable. A glycerol ester derivative may be any of a monoglyceride, a diglyceride or a triglyceride, although a diglyceride or a triglyceride is preferable, with a triglyceride being most preferable. In the case of a diglyceride or a triglyceride, the position at which the conjugated unsaturated fatty acid is esterified can be selected without particular limitation in accordance with the objective. Specific examples of conjugated unsaturated fatty acid ester derivatives include ethyl punicate, butyl punicate, propyl punicate, 1-punicyl-sn-glycerol, 2-punicyl-sn-glycerol, 1,2-dipunicyl-sn-glycerol, 2,3-dipunicyl-sn-glycerol and 1,2,3-tripunicyl-sn-glycerol.

Pomegranate seed oil can be used as the oil/fat composition having PPAR agonist activity of the present invention.

In the present invention, pomegranate seed oil can either be used as is, or processed into the form of fatty acids or derivatives thereof. A preferable method of processing into fatty acids is to subject the plant oil/fat to pretreatment if necessary, then hydrolyze to obtain the fatty acids, and then purify. Examples of pretreatment methods include physical methods such as a method in which the oil/fat is stood at a temperature above the melting point so that components having a high specific gravity precipitate and can be removed, and a method in which components having a low specific gravity are removed by centrifugal separation, and chemical methods such as a method in which sulfuric acid or phosphoric acid is added to the oil/fat and heating and stirring are carried out so as to decompose proteins and organic pigments, and then the decomposed products are removed by neutralizing and washing, and a method in which activated clay is added and heat treatment is carried out so as to adsorb and hence remove decomposed products, colored substances, resinous substances and the like. Moreover, specific examples of hydrolysis methods include chemical methods such as a method in which the oil/fat is saponified using an alkali such as potassium hydroxide, a medium pressure catalytic decomposition method in which decomposition is carried out under medium pressure conditions using zinc oxide, calcium oxide or magnesium oxide as a catalyst, and a continuous high pressure decomposition method in which continuous decomposition is carried out under high pressure, and biological hydrolysis methods using lipase, microorganisms or the like. Methods of separating and purifying fatty acids include a method in which the target fatty acids are purified by distillation using a batch type, semi-continuous type or continuous type distillation apparatus or a superfractionation apparatus, and a method in which a supersaturated solution or melt is cooled to a suitable temperature in accordance with the target fatty acids to produce crystals, and then the crystals are fractionated using a method such as a compression method, the Solexol method (US Patent No. 2293674, 1942), the Emersol method (US Patent No. 2421157, 1974) or the Henkel method (W. Stein et al., J. Am. Oil Chem. Soc., 45, 471, 1968).

Moreover, the conjugated unsaturated fatty acids that make up the active ingredient of the PPAR agonist of the present invention may also be prepared by culturing a microorganism such as an alga, extracting an oil or fat from the culture, and then obtaining the fatty acids therefrom using a publicly known method. Moreover, a conjugated unsaturated fatty acid may be prepared by reacting a publicly known unsaturated fatty acid in the presence of a chemical catalyst or using a microorganism, animal cells, or an enzyme extracted therefrom. An example is a method in which a highly conjugated unsaturated fatty acid having a conjugated trienoic structure is obtained from arachidonic acid, γ-linolenic acid or eicosapentaenoic acid using an isomerase produced by the red alga Ptilota filicina (M.L. Wise, Biochemistry, 33, 15223, 1994).

In the present invention, there are no particular limitations on the method of making a conjugated unsaturated fatty acid into an ester derivative. A publicly known method can be used, for example a direct esterification method in which an ester is synthesized from the fatty acid in question and an alcohol such as ethyl alcohol or glycerol by a dehydration reaction, a transesterification method in which an ester and an alcohol, an ester and the fatty acid in question, or an ester and an ester are reacted together to synthesize a new ester, a method in which an ester is synthesized from the chloride of the fatty acid in question and an alcohol, a method in which an epoxy compound and the fatty acid in question are reacted together, or a method in which an olefin and the fatty acid in question are reacted together; the direct esterification method and the transesterification method are, however, preferable. A specific example of the direct esterification method is a method in which the fatty acid in question and the alcohol are mixed together, if necessary an azeotropic dehydrating agent such as xylene is added, and then the mixture is heated in the presence of a catalyst such as sulfuric acid, p-toluenesulfonic acid, zinc chloride, activated alumina, titanium oxide or tetraisopropyl titanate. Moreover, specific examples of the transesterification method are a transesterification reaction between two fatty acid esters (Nenokichi Hirao, Discourse on Oil and Fat Chemistry, last volume, p522, publ.: Kazama Shobo, 1950 (in Japanese)), a reaction between a fatty acid ester and an alcohol (H.J. Wright et al., Oil & Soap, 21, 145, 1944), and a method by A.T. Gros, R.O. Feuge et al. in which an alkali is used as a catalyst (J. Am. Oil Chem., 26, 704, 1949).

The oil/fat composition having PPAR agonist activity of the present invention can be used as a food/beverage either as is or in a suitable blend. Moreover, the oil/fat composition can also be used as a raw material of any of various foods produced industrially or as a processed food ingredient. There are no particular limitations on the oil/fat composition content in the food/beverage, but this content is preferably 0.01 to 99wt%, more preferably 0.1 to 90wt%. Moreover, any of various carriers and additives allowed as foodstuffs can be blended in as desired. Any carriers or additives may be used, provided there are no adverse effects on the PPAR agonist activity of the oil/fat composition. Specific examples of carriers include extenders, diluents, fillers, dispersants, excipients such as glucose and lactose, binders such as hydroxypropylcellulose (HPC) and polyvinylpyrrolidone (PVP), solvents such as water, ethanol and plant oils, solubilizers, buffering agents such as bicarbonate of soda, dissolution accelerators, gelatinizing agents such as sodium CMC, HPMC, agar and gelatin, and suspending agents such as sodium CMC and sodium alginate. Specific examples of additives include seasonings for improving edibility or palatability such as monosodium glutamate and inosinic acid, flavorings such as vanilla, mint, rosemary, linalool and other natural flavorings, vitamins such as vitamin A, vitamin B1, vitamin B2, vitamin B6, vitamin C, vitamin E, pantothenic acid and nicotinic acid, sweeteners such as stevia, organic acids such as citric acid, malic acid, fumaric acid, malonic acid, succinic acid, tartaric acid and lactic acid, colorants, moisture preventing agents, fiber, electrolytes, minerals, nutrients, antioxidants, preservatives, aromatics, wetting agents, and natural plant extracts such as tea extract, coffee extract, cocoa extract, and extracts of fruits such as orange, grape, apple, peach, pineapple, pear, plum, cherry, papaya, tomato, melon, strawberry and raspberry.

A food/beverage containing the oil/fat composition of the present invention may take any form provided there are no adverse effects on the PPAR agonist activity of the oil/fat composition. Specific examples include coffee, tea beverages such as black tea, green tea and oolong tea, soymilk, fruit and vegetable beverages such as vegetable juices and fruit juices, lactic acid bacteria beverages such as yoghurt drinks, milk beverages such as cow's milk, carbonated beverages such as cola, various sports drinks, bakery products such as bread, rice, noodles, processed soybean products such as tofu, processed fish and meat foods such as sausages and ham, cakes, confectionery such as cookies, bean-jam buns (*manju*), rice crackers, ice cream, puddings, bean-jam jelly (*yokan*), candy and chocolate, dairy products such as butter, yoghurt and cheese, processed oil/fat foods such as margarine and shortening, condiments such as mayonnaise, dressings, miso, soy sauce and other sauces, arum root paste (*konnyaku*), and pickles.

A food/beverage containing the oil/fat composition of the present invention can be used as a food/beverage exhibiting health maintenance, health improvement or physical fitness improvement effects. Specifically, the food/beverage containing the oil/fat composition of the present invention can be used as a food/beverage producing effects such as improvement or suppression of obesity, reduction of accumulated fat, in particular visceral fat, suppression of fat accumulation, in particular visceral fat accumulation, improvement of lipid metabolism abnormalities such as a tendency toward hyperlipidemia or a tendency toward high blood cholesterol, improvement of glucose metabolism abnormalities such as a tendency toward diabetes or a tendency toward high blood glucose after meals, improvement of a tendency toward hypertension, and suppression of thickening of the artery walls, or as a therapeutic food/beverage during cancer treatment.

When used as an active ingredient of a medicine, the oil/fat composition having PPAR agonist activity of the present invention is administered to a human or other mammal either as is or else with 0.01 to 99.5wt%, preferably 0.1 to 90wt%, thereof contained in a medicinally accepted non-toxic inactive carrier. The amount of the medicine administered to the human or other mammal can be selected as appropriate in accordance with the objective, provided this amount is within a range such that the target effect is produced but there is no toxicity. However, it is preferable to administer an effective dose such that the intake of the oil/fat composition is in a range of 1mg to 3g per 1kg of body weight either once per day or else in a plurality of divided doses per day. The exact dose of the medicine may vary within a broad range depending on the method of administration, the formulation of the medicine, the medical condition and body weight of the subject being treated, and the like, and is thus preferably determined through the experience and judgement of a responsible doctor or veterinarian. Moreover, there are no particular limitations on the carrier, with it being possible to use any carrier commonly used when manufacturing a solid, semi-solid or liquid medicinal preparation. Specifically, one or more selected from excipients such as glucose and lactose, disintegrators such as starch and carboxymethylcellulose-calcium (CMC-Ca), binders such as hydroxypropylcellulose (HPC) and polyvinylpyrrolidone (PVP), lubricants such as talc and magnesium stearate, pH regulators such as bicarbonate of soda, stabilizers, diluents, pigments, other prescription-use auxiliary agents, and the like can be used. It is generally preferable for a medicine to be administered in a unit dosage form, and the medicine of the present invention can be administered orally as a tablet, a capsule, granules, a powder, a sugar-coated tablet, a suspension, a solution, a syrup, drops, a sublingual tablet, an emulsion or the like, or non-orally as an injection, a suppository or the like. When prolonged administration is required, oral administration is preferable.

The medicine containing the oil/fat composition of the present invention can be used as an anti-obesity agent, a visceral fat reducing agent, a visceral fat accumulation suppressing agent, a hyperlipidemia treatment agent, a high blood cholesterol level treatment agent.

The oil/fat composition of the present invention can be used as an animal feed either as is or in a suitable blend with seasonings, aromatics and the like to improve edibility and palatability. In this case, to maintain constant physical properties, emulsifiers and stabilizers can also be blended in. Moreover, the oil/fat composition of the present invention can also be used as a raw material of any of various processed animal feeds and pet foods produced industrially. Furthermore, the oil/fat composition of the present invention may also be used sprinkled directly onto an animal feed. There are no particular limitations on the oil/fat composition content in the animal food, but this content is, for example, in a range of 0.1 to 99.5wt%, preferably 0.5 to 90wt% relative to the animal feed as a whole in terms of solids. The animal feed can be used for preventing or improving obesity, preventing or treating diabetes, or preventing or treating cancer, in livestock or pets.

Moreover, when the oil/fat composition of the present invention is used in a food/beverage, medicine or animal feed, it may be used in combination with other food/beverage, medicinal or animal feed compositions that have similar effects.

### BEST MODE FOR CARRYING OUT THE INVENTION

Following is a more detailed description of the present invention through examples, although it should be noted that the present invention is not limited to these examples.

### (Example 1) Preparation of conjugated unsaturated fatty acid glycerol esters and conjugated unsaturated fatty acids from plant seeds

Pomegranate seeds obtained by splitting open pomegranates (produce of California, USA) purchased from a fruit shop and then removing the sap fruit from around the seeds and drying the seeds, marigold seeds, karela seeds and balsam seeds purchased from a seed merchant, and catalpa seeds purchased from a herbal medicine shop were each pulverized, and then double the weight of n-hexane was added and extraction was carried out twice at room temperature for 6 hours, thus obtaining liquid extracts from each of the seed types. The solvent was removed from each of the liquid extracts, thus obtaining pomegranate seed oil, marigold seed oil, karela seed oil, and catalpa seed oil. When a hexane solution of each of these seed oils and also commercially sold tung oil (Nacalai Tesque) was spotted onto a silica gel 60F254 TLC plate (made by E. Merck), developing was carried out using a 60:40:1 mixture of n-hexane, ethyl ether and acetic acid, and then coloring was carried out using iodine, a principal spot corresponding to triglycerides from Rf 0.5 to 0.7 as with tung oil was observed. 1g of each of the plant seed oils obtained as above and tung oil was dissolved in ethanol, and then KOH was added and heating carried out to cause hydrolysis. Unsaponifiable matter was then removed from the hydrolysis products, the aqueous phase was separated off and adjusted to pH 2.5, and then n-hexane extraction was carried out before removing the solvent under reduced pressure, thus obtaining free fatty acids. Each of the fatty acid fractions was subjected to TLC analysis as above, whereupon it was found that the principal components were free fatty acids. The free fatty acids thus obtained were subjected to silica gel column chromatography (Wakogel C-200 made by Wako Pure Chemical Industries), washing with n-hexane was carried out, and then elution was carried out using n-hexane containing 20vol% ethyl ether, thus obtaining purified fatty acid fractions. The yields were 0.88g from the pomegranate seed oil, 0.91g from the marigold seed oil, 0.92g from the tung oil, 0.90g from the karela seed oil, 0.90g from the catalpa seed oil, and 0.89g from the balsam seed oil.

The fatty acids obtained as above and commercially sold CLA (made by Kenko Tsusho) were each treated with a sulfuric acid - methanol (2:230 by volume) solution in the presence of dimethylsulfoxide to form fatty acid methyl ester derivatives, and then analysis was carried out by gas chromatography under the following conditions.
System: HP (Hewlett-Packard) 5890 Series II
Column: SPELUCO SP-2380, 100mm×0.25mm ID
Column temperature: 185ºC
Injection temperature: 200ºC
Detector temperature: 210ºC

Moreover, following Takagi's method (Takagi, Nihon Kagaku Zasshi, 86, 296, 1965 (in Japanese)), 60ml of ethanol and 10ml of hydrazine hydrate were added to 0.5g of each of the fatty acids, a cooler was attached, mixing was carried out for 2 hours while blowing in air at 50ºC, then the reaction products were extracted with diethyl ether, the extract was washed with an aqueous hydrochloric acid solution, and then the ether was removed. 10ml of methanol containing 2% sulfuric acid was added to the products, and the mixture was left overnight under nitrogen, thus obtaining methyl esters. The products thus obtained were analyzed using silver nitrate immersed silica gel thin film chromatography. Moreover, the disulfide dimethyl adducts were produced, and were analyzed using GC/MS (ThermoQuest GC-9).

Based on the results of the analyses, the number of carbon atoms, the number of unsaturated bonds, the positions of the unsaturated bonds and the stereoisomerism were determined for the fatty acids. The results are shown in Table 1.

**Table 1: Compositions of fatty acids from plant seed oils**

| Fatty acid | Structure | Content out of all fatty acids (wt%) | | | | | | |
|---|---|---|---|---|---|---|---|---|
| | | Fatty acids from marigold seed oil | Fatty acids from pomegranate seed oil | Fatty acids from catalpa seed oil | Fatty acids from karela seed oil | Fatty acids from tung oil | Fatty acids from balsam seed oil | CLA |
| Palmitic acid | C 16:0 | 4.0 | 2.9 | 3.0 | 1. 6 | 2.5 | 4.6 | 6.9 |
| Stearic acid | C 18:0 | 2.1 | 2.1 | 1.9 | 17.3 | 2.6 | 3.0 | 2.5 |
| Oleic acid | | C 18:1 7.4 | 3.9 | 7.8 | 12.1 | 5.9 | 13.4 | 18.1 |
| Linoleic acid | C 18:2 | 42.6 | 4.2 | 32.3 | 7.3 | 6.5 | 9.4 | 0.7 |
| Linolenic acid | C 18:3 | 0.4 | ND | 1.3 | 0.4 | 0.9 | 17.7 | 0.1 |
| Conjugated linoleic acid | C 18:2 | ND* | ND | ND | ND | ND | ND | 67.2 |
| Calendic acid | C 18:3 | 33.4 | ND | ND | ND | ND | ND | ND |
| Punicic acid | C 18:3 9c, 11,13c | 0.1 | 71.6 | 0.5 | ND | ND | ND | ND |
| Catalpic acid | C 18:3 9c,11t,13t | ND | 5.1 | 31.3 | ND | 1.8 | ND | ND |
| α-eleostearic acid | C 18:3 9c,11t,13t | 0.4 | ND | 0.5 | 46.5 | 70.4 | ND | ND |
| β-eleostearic acid | C 18:3 9t,11t,13t | ND | 1.6 | 5.7 | ND | 6.2 | ND | ND |
| Parinaric acid | C 18:4 9c,11t,13t,15c | ND | ND | ND | ND | ND | 48.0 | ND |

| | | | | | | | | |
|---|---|---|---|---|---|---|---|---|
| ND* = not detected (i.e. below detection limit) | | | | | | | | |

It can be seen that the principal component of the fatty acids from pomegranate seed oil is punicic acid, the principal components of the fatty acids from marigold seed oil are calendic acid and linoleic acid, the principal component of the fatty acids from tung oil and from karela seed oil is α-eleostearic acid, the principal components of the fatty acids from catalpa seed oil are catalpic acid and linoleic acid, and the principal component of the fatty acids from balsam seed oil is parinaric acid.

### (Example 2) PPAR agonist activity of fatty acids from plant seed oils

2×10⁴ cells of CV-1 (cultured cells originating from the kidney of a male African green monkey, bought from Human Sciences Foundation) were planted in a 24-well plate into which 500µl of DMEM (Dulbecco's Modified Eagle Medium by Gibco) containing 10% bovine fetal serum (made by Gibco), penicillin and streptomycin (10000 units/ml of each, 10000µg/ml, made by Gibco) and ascorbic acid (37µg/ml, made by Wako Pure Chemical Industries) had been put, culturing was carried out for 24 hours under conditions of 37ºC and 5% CO₂, washing was carried out with OPTI-MEM (made by Gibco), and then pM-PPAR [a chimeric-protein-expressing plasmid prepared by inserting a PPARγ ligand binding site gene (amino acid sequence 204-505) into the C-terminal side of GAL4 of a pSV40 expression vector containing a yeast-derived transcription factor GAL4 gene (amino acid sequence 1-147)] which had been prepared and stored in advance following Yoshikawa's method (Kazuaki Yoshikawa, Gene Introduction/Expression Research Methods for Neurophysiology, Springer-Verlag Tokyo, 1997 (in Japanese)) and 4×UASg-luc [a reporter plasmid into which a GAL4 responsive sequence (UASg) has been introduced 4 times upstream of a luciferase gene, which is a reporter gene], or pM [a plasmid in which the PPARγ ligand binding site sequence has been removed from above-mentioned pM-PPAR] and 4×UASg-luc, were transfected using Lipofect Amine (made by Gibco), and 5 hours after the transfection had been completed DMEM containing 20% bovine fetal serum was added, the serum concentration was adjusted to 10%, and culturing was carried out overnight. The next morning, ligand-reagent-added groups were established by exchanging with culture media prepared by drying up under a stream of nitrogen a hexane solution of conjugated linoleic acid (CLA, purchased from Kenko Tsusho) or fatty acids from pomegranate seed oil, fatty acids from marigold seed oil, fatty acids from tung oil, fatty acids from catalpa seed oil, fatty acids from karela seed oil or fatty acids from balsam seed oil prepared as in Example 1, dissolving in dimethylsulfoxide (DMSO), and diluting by a factor of 1000 using DMEM containing 10% activated-charcoal-treated bovine fetal serum (final fatty acid concentration 20 µM). Moreover, untreated control groups were established by exchanging with a culture medium prepared by diluting only DMSO with DMEM containing 10% activated-charcoal-treated bovine fetal serum. Furthermore, positive control groups were established by exchanging with a culture medium containing troglitazone(made by Sankyo, hereinafter abbreviated to 'TZD') (1µM), which is conventionally known as a strong PPARγ agonist. One day after the culture medium exchange, the culture medium was removed, the cells were washed with phosphate buffered saline (PBS), a cell solution was prepared using PicaGene cell solution Luc (made by Toyo Ink), 20µl of the cell solution was reacted with 100µl of a PicaGene luminescent substrate (made by Toyo Ink), and the luciferase activity was measured using a luminometer (Berthold Lumat LB9501) with the measurement time set to 10 seconds.

The results obtained were analyzed as follows. Letting the ratio of the measurement group (the group for which pM-PPAR and 4×UASg-luc were transfected) to the control group (the group for which pM and 4×UASg-luc were transfected) in the case of the untreated control groups be (a), and the ratio of the measurement group to the control group in the case of the ligand-reagent-added groups be (b), the PPARγ agonist activity caused by the ligand reagent was evaluated using the ratio (b)/(a). To avoid errors due to differences in the transfection efficiency between experiments, TZD (1µm) was put in as positive control groups and the values between the experiments were corrected.

**Table 2: PPAR agonist activities of conjugated unsaturated fatty acids from plant seed oils**

| Additive | Concentration | Relative activity (mean, n=3) |
|---|---|---|
| Untreated control group | none | 100 |
| Positive control group (TZD) | 1 *µ*M | 329 |
| CLA | 20 *µ*M | 125 |
| Fatty acids from pomegranate seed oil | 20 *µ*M | 187 |
| Fatty acids from marigold seed oil | 20 *µ*M | 209 |
| Fatty acids from tung oil | 20 *µ*M | 164 |
| Fatty acids from karela seed oil | 20 *µ*M | 158 |
| Fatty acids from catalpa seed oil | 20 µM | 140 |
| Fatty acids from balsam seed oil | 20 µM | 170 |

As shown in Table 2, all of the groups for which plant-seed-derived fatty acids having conjugated trienoic acids as principal components were added were found to have a stronger PPAR agonist activity than CLA. A strong PPAR agonist activity better than that of CLA was not observed for fatty acids other than the conjugated trienoic acids contained in the fatty acid fractions, and hence it is clear that the conjugated trienoic acids have a strong PPAR agonist activity.

### (Example 3) Comparison of PPAR agonist activity between fatty acids from pomegranate seed oil and CLA

PPAR agonist activity was evaluated for CLA and fatty acids from pomegranate seed oil as in Example 2, only the concentrations of the CLA and the fatty acids from pomegranate seed oil were varied. The results are shown in Table 3. It can be seen that the fatty acids from pomegranate seed oil exhibit about the same PPARγ agonist activity as 10 times the concentration of CLA, and that at a concentration of 57µM or more the fatty acids from pomegranate seed oil exhibit about the same activity as 1µM of TZD.

**Table 3: PPAR agonist activity of fatty acids from pomegranate seed oil and CLA**

| Sample | Concentration (µm) | Relative activity (n=3) |
|---|---|---|
| Untreated control | none | 100 |
| Positive control (TZD) | 1 | 450 |
| CLA | 3 | 129 |
| | 9 | 109 |
| | 30 | 110 |
| | 90 | 159 |
| | 300 | 323 |
| Fatty acids from pomegranate seed oil | 6 | 105 |
| | 10 | 141 |
| | 18 | 209 |
| | 32 | 246 |
| | 57 | 419 |
| | 100 | 400 |

### (Example 4) Visceral fat reduction effects and lipid metabolism amelioration effects of pomegranate seed oil

10-week old female C57BL/6J mice (from Clea Japan) were made obese by keeping for 3 weeks on a high-fat, high-sugar feed (made by Oriental Yeast) having the composition shown in Table 4. The mice were then divided into groups of 8, and were kept for a further 3 weeks on a test feed consisting of a fat-free growth-period-use standard mixed feed (AIN-93G, made by Oriental Yeast) with 2wt% soybean oil as an essential fatty acid source and either 7wt% of a test oil or 7wt% of a control oil added. After 3 weeks, the mice were fed for the last time and then not fed for 16 hours, after which the whole blood was collected from the mice using a heparinized syringe under ether anesthesia, and then the mice were dissected, with the adipose tissue around the kidneys, the adipose tissue around the ovaries, the liver, the kidneys and the spleen being extracted, and the weights thereof being measured. Each organ weight thus obtained was divided by the body weight to obtain the ratio of the organ weight to the body weight. The adipose tissue around the kidneys and the adipose tissue around the ovaries was evaluated as visceral adipose tissue. The collected blood was centrifuged for 15 minutes at 3000 rpm while cooling to obtain the plasma, and then the total cholesterol concentration was measured using Cholesterol C Test Wako (made by Wako Pure Chemical Industries), the neutral lipid concentration using Triglyceride G Test Wako (made by Wako Pure Chemical Industries), and the free fatty acid concentration using NEFA C Test Wako (made by Wako Pure Chemical Industries). The groups set up were a test group for which pomegranate seed oil prepared as in Example 1 was added to the feed (pomegranate seed oil group), a control group for which soybean oil was added to the feed (soybean oil group), and a comparative group for which high-DHA-containing fish oil (DHA content 20wt%, made by Harima Chemicals), which is known to have obesity ameliorating effects, was added to the feed (fish oil group). Also set up was a group for which 6.5wt% of soybean oil and 0.5wt% of CLA (made by Kenko Tsusho, 70% purity) was added to the feed (CLA group). According to the results of preliminary tests, if 1wt% or more of CLA is added to the feed then marked liver enlargement (1.4 times relative to a control) occurs and there is a drop in the mice's food intake; it was thus judged that a substantial dose is 0.5wt% or less, and hence 0.5wt% was decided upon. Moreover, also set up was a group that were fed a standard feed containing 9wt% of soybean oil throughout the test period right from the start (standard feed group, n=3). During the test period, the mice were allowed to drink water freely, but feeding was limited such that the intake was the same as for the soybean oil group.

**Table 4: Composition of high-fat, high-sugar feed**

| Component | Content (wt%) | Component | Content (wt%) |
|---|---|---|---|
| Casein | 25.000 | AIN-93 mineral mixture | 3.500 |
| Corn starch | 14.869 | AIN-93 vitamin mixture | 1.000 |
| Sucrose | 20.000 | Choline bitartrate | 0.250 |
| Soybean oil | 15.000 | Tert-butylhydroquin one | 0.006 |
| Lard | 15.000 | L-cystine | 0.375 |
| Cellulose powder | 5.000 | | |
| Energy ratio: fat 53%, carbohydrate 27%, protein 20% Total energy: 5100Kcal/kg | | | |

Turning to the results, during the test period no significant differences were found in food intake or body weight increase between the groups. However, as shown in Table 5, the ratio of the visceral fat weight to the body weight clearly increased for the soybean oil group relative to the standard feed group, suggesting that the mice exhibited visceral fat type obesity due to the intake of the high-fat high-sugar feed. Comparing the ratio of the visceral fat weight to the body weight for each of the groups, the order is fish oil group > soybean oil group > CLA group > pomegranate seed oil group, clearly showing that pomegranate seed oil has better visceral fat reduction effects than fish oil or CLA.

**Table 5: Visceral fat reduction effects of pomegranate seed oil**

| | Ratio of tissue weight to body weight (mean, n=8) | | | | | |
|---|---|---|---|---|---|---|
| Group | Visceral fat | Fat around kidneys | Fat around ovaries | Liver | Kidneys | Spleen |
| Standard feed group | 0.891 | 0.238 | 0.653 | NT | NT | NT |
| Soybean oil group | 1.144 | 0.304 | 0.839 | 4.454 | 1.128 | 0.391 |
| Fish oil group | 1.480* | 0.520* | 0.960 | 5.160* | 1.300* | 0.460* |
| CLA group | 0.890 | 0.240 | 0.650 | 4.920 | 1.280 | 0.450 |
| Pomegranate seed oil group | 0.760* | 0.210 | 0.560* | 5.410 | 1.260 | 0.390 |

| | | | | | | |
|---|---|---|---|---|---|---|
| NT: Not tested | | | | | | |
| *: P<0.05 | | | | | | |

Moreover, the results of analyzing the total cholesterol concentration, neutral lipid concentration and free fatty acid concentration in the plasma are shown in Table 6. With the pomegranate seed oil, a tendency for neutral lipid reduction and a statistically significant free fatty acid reduction effect were seen, and thus a lipid metabolism amelioration effect in vivo was seen.

**Table 6: Lipid metabolism amelioration effects of pomegranate seed oil**

| | Relative blood lipid concentration (mean, n=8) | | |
|---|---|---|---|
| Group | Total cholesterol (mg/dl) | Neutral lipids (mg/dl) | Free fatty acids acids (mEq/L) |
| Soybean oil group | 100 | 100 | 100 |
| Fish oil group | 57** | 72 | 40** |
| CLA group | 89 | 100 | 71* |
| Pomegranate seed oil group | 105 | 72 | 52** |

| | | | |
|---|---|---|---|
| NT: Not tested | | | |
| *: P<0.05, **: P<0.01 | | | |

### (Example 5) Visceral fat accumulation suppression effects of pomegranate seed oil

6-week old female ICR type CD-1 mice (from Charles River) were divided into groups of 8, and were kept for 4 weeks using a feed prepared by adding a prescribed amount of a test oil to a fat-free mouse/rat standard mixed feed (growth-period-use AIN-93G, made by Oriental Yeast). After 4 weeks the mice were dissected under ether anesthesia, with the adipose tissue around the kidneys, the adipose tissue around the ovaries, the liver, the kidneys and the spleen being extracted, and the weights thereof being measured. The sum of the weights of the adipose tissue around the kidneys and the adipose tissue around the ovaries was used to evaluate visceral fat. The test groups set up were a group for which 1.7wt% (1wt% in terms of punicic acid) of pomegranate seed oil prepared as in Example 1 and 5.3wt% of soybean oil were added to the feed (pomegranate seed oil group), a control group for which 7wt% of soybean oil was added to the feed (soybean oil group), and a comparative group for which 7wt% of high-DHA-containing fish oil (DHA content 20wt%, made by Harima Chemicals), which is known to have obesity ameliorating effects, was added to the feed (fish oil group).

Turning to the results, during the test period no significant differences were found in food intake or body weight increase between the groups. The measurement results for the weights of the various organs are shown in Table 7 as the ratio of the organ weight to the body weight. A statistically significant visceral fat accumulation suppression effect was found with the fish oil group. A visceral fat accumulation suppression effect almost equal to that of the fish oil was found with the pomegranate seed oil group.

**Table 7: Visceral fat accumulation suppression effects of pomegranate seed oil**

| | Ratio of tissue weight to body weight (mean, n=8) | | | | | |
|---|---|---|---|---|---|---|
| Group | Visceral fat | Fat around kidneys | Fat around ovaries | Liver | Kidneys | Spleen |
| Soybean oil group | 6.75 | 2.40 | 4.35 | 4.75 | 1.00 | 0.45 |
| Fish oil group | 5.35* | 1.95* | 3.40* | 4.92 | 1.01 | 0.46 |
| Pomegranate seed oil group | 5.56 | 2.02 | 3.54 | 4.82 | 1.02 | 0.44 |

| | | | | | | |
|---|---|---|---|---|---|---|
| *: P<0.05 | | | | | | |

### (Example 6) Cancer cell proliferation suppression effects of highly conjugated unsaturated fatty acids (not part of the claimed invention).

200µl of an RPMI-1640 culture medium with 10% bovine fetal serum added (made by Gibco BRL) in which DLD-1 cells from human colon cancer (5×10⁵cells/ml) had been suspended was put into each well of a 96-well plate and culturing was carried out for 24 hours under conditions of 37ºC and 5% CO₂. A fatty acid solution prepared in advance by taking a hexane solution of either fatty acids from pomegranate seed oil or fatty acids from tung oil prepared as in Example 1, sufficiently vaporizing the solvent under a stream of nitrogen, dissolving in dimethylsulfoxide and then diluting with phosphate buffered saline (PBS) was then added to each well, and culturing was carried out for a further 20 hours. 1µci/10µl/well of [3H]thymidine (made by Amersham Japan) was then added, and culturing was carried out for a further 4 hours. The cells were washed with PBS, then dissolved in 100µl of a 2N NaOH solution, and neutralized with 50µl of 4N HCl, and then the radioactivity was measured using a liquid scintillation counter. An experiment in which PBS was used instead of the test sample was used as a control. The results are shown in Table 8. It was found that both tung oil and pomegranate seed oil have cancer cell proliferation suppression effects.

**Table 8: Cancer cell proliferation suppression effects of fatty acids from tung oil and fatty acids from pomegranate seed oil**

| Additive | Relative [3H] thymidine uptake per well (mean, n=3) |
|---|---|
| Control | 100 |
| Fatty acid fraction from tung oil | 20 |
| Fatty acid fraction from pomegranate seed oil | 45 |

### (Example 7) Evaluation of safety of pomegranate seed oil

Five 6-week old female ICR type CD-1 mice (from Charles River) were forcibly orally administered 10g/kg of pomegranate seed oil, and the general state of the mice was observed for 1 week. As a result, no particular toxic symptoms were found. Moreover, groups of 8 of the above mice were kept for 4 weeks on a fat-free mouse/rat standard mixed feed (growth-period-use AIN-93G, made by Oriental Yeast) with 2wt% of soybean oil and 7wt% of pomegranate seed oil prepared as in Example 1 added (pomegranate seed oil group), or 8.5wt% of soybean oil and 0.5wt% of fatty acids from pomegranate seed oil prepared as in Example 1 added (pomegranate seed oil derived fatty acid group), or 9wt% of soybean oil added (soybean oil group), and changes in body weight, changes in food intake, and the general state of the mice were observed, and also the weights of major tissues after the 4 weeks were measured. As a result, it was found that there was no difference in body weight change or food intake between the groups, and no particular signs of toxicity. (Example 8) Manufacture of margarine containing pomegranate seed oil

80 parts by weight of a fat composition consisting of 60 wt% of hardened soybean oil (melting point 40ºC), 20wt% of palm oil and 20wt% of corn oil, 0.3 parts by weight of lecithin, 0.3 parts by weight of monoglyceride, 16 parts by weight of water, 2 parts by weight of common salt, 5 parts by weight of pomegranate seed oil prepared as in Example 1, and a small amount of vitamin E as an antioxidant were emulsified for 15 minutes at 60ºC using a TK homomixer at 50V while blowing in nitrogen gas, and then kneading was carried out while cooling rapidly to 15ºC, thus obtaining a sheet-shaped margarine that had no problems in terms of taste.

### (Example 9) Manufacture of a pie using margarine containing pomegranate seed oil

1250g of the sheet-shaped margarine containing pomegranate seed oil obtained in Example 8, 1500g of weak flour, 1000g of strong flour and 60g of dry milk were mixed together, an aqueous solution prepared by dissolving 45g of table salt and 1500g of sugar in 1250ml of water was added thereto, the mixture was gently mixed to produce a dough, the dough was stretched out into a sheet shape, shaping was carried out by folding into three 5 times, and baking was carried out in an oven, thus producing a pie.

### (Example 10) Manufacture of bread containing fatty acids from pomegranate seed oil

When mixing together bakery ingredients (100 parts by weight of strong wheat flour, 3 parts by weight of yeast, 3 parts by weight of dry milk, 5 parts by weight of sugar, 70 parts by weight of water, 2 parts by weight of salt, and 7 parts by weight of margarine), 1 part by weight of fatty acids from pomegranate seed oil obtained as in Example 1 was added per 100 parts by weight of the wheat flour. The mixture was kneaded and allowed to ferment to produce a bread dough, the bread dough was taken¹ a floor time for 60 minutes at 25ºC, divided into portions, taken² a bench time for 30 minutes at room temperature, then put into bread molds, left for 60 minutes at a temperature of 38ºC and a humidity of 90%, and then baked in an oven (45 minutes at 190ºC), thus producing bread.
¹ ???
² ???

### (Example 11) Manufacture of chocolate containing pomegranate seed oil

22 parts by weight of cacao mass, 10 parts by weight of cacao butter, 10 parts by weight of alternative cacao batter fat, 8 parts by weight of whole milk powder, and 1 part by weight of pomegranate seed oil prepared as in Example 1 were mixed together well at 60ºC, the mixture was passed through a refiner, conching and tempering were carried out, and then the mixture was poured into molds and cooled, thus producing chocolate bars.

### (Example 12) Manufacture of whipping cream containing pomegranate seed oil

0.8 parts by weight of synthesized diglycerol stearate as an emulsifier, 0.6 parts by weight of soy lecithin and 3 parts by weight of pomegranate seed oil were added to a mixed oil consisting of 70 parts by weight of hardened rapeseed oil having a slipping point of 34ºC and 30 parts by weight of hardened coconut oil having a slipping point of 32ºC while melting at an oil temperature of 70ºC, thus producing an oil composition. Separate to this, 0.1 parts by weight of sodium hexametaphosphate, was added to 54.9 parts by weight of skimmed milk, and the skimmed milk was heated to 55ºC while stirring. 45 parts by weight of the above-mentioned emulsifier-containing oil composition was then added to the skimmed milk, preliminary emulsification was carried out while holding the temperature at 65ºC, the mixture was passed through a homogenizer and homogenization was carried out first at a pressure of 80kg/cm² and then at a pressure of 20kg/cm², then sterilization was carried out for 15 seconds at 95ºC, the mixture was cooled to 5ºC using a plate type cooler, and then aging was carried out for 24 hours in an incubator at 5ºC, thus obtaining a whippable synthetic cream. There was no problem with the taste of the cream obtained.

### (Example 13) Manufacture of French dressing containing pomegranate seed oil

150 parts by weight of salad oil, 30 parts by weight of pomegranate seed oil, 100 parts by weight of wine vinegar, and small amounts of salt and pepper were mixed together, thus producing a French dressing, which had no problems in terms of taste.

### (Example 14) Manufacture of beverage containing conjugated unsaturated fatty acids or triglycerides thereof

1 part by weight of pomegranate seed oil, karela seed oil, marigold seed oil, balsam seed oil, fatty acids from pomegranate seed oil, fatty acids from tung oil, fatty acids from marigold seed oil or fatty acids from balsam seed oil prepared as in Example 1, 8.5 parts by weight of a mixed emulsifier, and 90.5 parts by weight of a reduced starch saccharified substance were mixed together to produce an emulsion, and then sterilization was immediately carried out by heating to 80ºC for 10 minutes. 5 parts by weight of the emulsion was then mixed together with 5 parts by weight of granulated sugar, 14 parts by weight of liquid sugar, 1 part by weight of aromatics, 0.5 parts by weight of citric acid, 0.2 parts by weight of sodium citrate, and 74 parts by weight of water, thus obtaining a beverage having a pH of 3.3.

### (Example 15) Preparation of capsule for oral administration

40mg of pomegranate seed oil, karela seed oil, marigold seed oil or balsam seed oil obtained as in Example 1 or commercially sold tung oil, 200mg of lactose, 70mg of starch, 50mg of polyvinylpyrrolidone and 35mg of crystalline cellulose were mixed together, and then the mixture was filled into a #3 gelatin capsule, and the surface was gelatin coated, thus preparing a capsule for oral administration.

### (Example 16) Preparation of tablets for oral administration

5g of fatty acids from pomegranate seed oil, fatty acids from tung oil, fatty acids from marigold seed oil or fatty acids from balsam seed oil prepared as in Example 1, 70g of lactose and 30g of corn starch were mixed together into a uniform mixture, and then 25ml of a 10% hydroxypropylcellulose solution was added thereto, and stirring was carried out and granules formed. The granules were dried and graded, 2g of magnesium stearate and 2g of talc were added, mixing was carried out, and tablets were produced using a rotary tablet machine.

### (Example 17) Preparation of emulsion containing conjugated trienoic acid triglycerides

5 parts by weight of pomegranate seed oil, marigold seed oil, catalpa seed oil, karela seed oil or balsam seed oil obtained as in Example 1 or tung oil was added to 95 parts by weight of Intralipid made by Baxter and emulsification was carried out, thus obtaining an emulsion containing conjugated trienoic acid triglycerides. (Example 18) Preparation of pet food containing conjugated unsaturated fatty acids or triglycerides thereof

80 parts by weight of chicken paste, 10 parts by weight of ground red meat of beef, 10 parts by weight of soy protein, 3 parts by weight of pomegranate seed oil, karela seed oil, marigold seed oil, balsam seed oil prepared as in Example 1, fatty acids from pomegranate seed oil, fatty acids from tung oil, fatty acids from marigold seed oil or fatty acids from balsam seed oil prepared as in Example 1, 1 part by weight of chemical seasonings, 1 part by weight of tocopherol, and small amounts of calcium, vitamins, starch and sorbitol were kneaded together using a food cutter, filled into sheep intestines, cooked by heating at 90 to 95ºC, and dried in a current of air at 50ºC, thus obtaining a dry sausage type pet food.

### INDUSTRIAL APPLICABILITY

The oil/fat composition containing conjugated trienoic acids or conjugated tetraenoic acids exhibiting PPAR agonist activity or salts or ester derivatives thereof exhibits visceral fat reduction effects, visceral fat accumulation suppression effects, lipid metabolism abnormality amelioration effects, glucose metabolism abnormality amelioration effects and cancer cell proliferation suppression effects, and can thus be used as a health beverage, health food, supplementary food or medicinal composition with an objective of improving diet or tendencies toward the above-mentioned physical disorders in humans, and can also be used as an ingredient of an animal feed for preventing or treating tendencies toward obesity or diabetes or an animal feed for preventing or treating cancer in pets.

## Claims

1. Use of an agonist comprising at least one selected from punicic acid which is a conjugated unsaturated fatty acid having 18 carbon atoms and containing a conjugated trienoic structure (-CH=CH-CH=CH-CH=CH-) in the molecule thereof, and salts and ester derivatives thereof for the preparation of a pharmaceutical composition for reducing visceral fat amount, suppressing visceral fat accumulation, or preventing or ameliorating a lipid metabolism abnormality in a human or animal by promoting binding of a peroxisome proliferator-activated receptor to a target gene sequence and promoting gene expression downstream thereof in a human or animal.

2. The use of the agonist according to claim 1, wherein said punicic acid is extracted from pomegranate seed oil.

3. Use of pomegranate seed oil for the preparation of a pharmaceutical composition for reducing visceral fat amount, suppressing visceral fat accumulation, or preventing or ameliorating a lipid metabolism abnormality in a human or animal by promoting binding of a peroxisome proliferator-activated receptor to a target gene sequence and promoting gene expression downstream thereof in a human or animal.

## Patentansprüche

1. Verwendung eines Agonisten, umfassend mindestens einen ausgewählt aus Punicinsäure, die eine konjugierte ungesättigte Fettsäure mit 18 Kohlenstoffatomen ist und eine konjugierte Trien-Struktur (-CH=CH-CH=CH-CH=CH-) in dem Molekül enthält, und Salzen und Esterderivaten davon, für die Herstellung eines Arzneimittels zur Verminderung der Menge an Bauchfett, zur Unterdrückung der Anreicherung von Bauchfett, oder zur Verhinderung oder Verbesserung einer Lipidstoffwechselabnormalität in einem Menschen oder Tier durch die Förderung der Bindung eines Peroxisom-Proliferator-aktivierenden Rezeptors an eine Zielgensequenz und Förderung der nachgeordneten Genexpression in einem Menschen oder Tier.

2. Verwendung des Agonisten nach Anspruch 1, wobei die Punicinsäure aus Granatapfelsamenöl extrahiert wird.

3. Verwendung von Granatapfelsamenöl für die Herstellung eines Arzneimittels zur Verminderung der Menge an Bauchfett, zur Unterdrückung der Anreicherung von Bauchfett, oder zur Verhinderung oder Verbesserung einer Lipidstoffwechselabnormalität in einem Menschen oder Tier durch die Förderung der Bindung eines Peroxisom-Proliferator-aktivierenden Rezeptors an eine Zielgensequenz und Förderung der nachgeordneten Genexpression in einem Menschen oder Tier.

## Revendications

1. Utilisation d'un agoniste comprenant au moins un choisi parmi un acide punicique qui est un acide gras insaturé conjugué ayant 18 atomes de carbone et contenant une structure triénoïque conjuguée (-CH=CH-CH=CH-CH=CH-) dans sa molécule, et ses sels et dérivés esters pour la préparation d'une composition pharmaceutique pour réduire la quantité de graisse viscérale, supprimer l'accumulation de graisse viscérale ou prévenir ou améliorer une anormalité du métabolisme lipidique chez un humain ou un animal en favorisant la liaison d'un récepteur activé par les proliférateurs de péroxisomes à une séquence de gène cible et en favorisant l'expression génique en aval de celle-ci chez un humain ou un animal.

2. Utilisation de l'agoniste selon la revendication 1, dans laquelle ledit acide punicique est extrait de l'huile de graine de grenade.

3. Utilisation d'huile de graine de grenade pour la préparation d'une composition pharmaceutique pour réduire la quantité de graisse viscérale, supprimer l'accumulation de graisse viscérale ou prévenir ou améliorer une anormalité du métabolisme lipidique chez un humain ou un animal en favorisant la liaison d'un récepteur activé par les proliférateurs de péroxisomes à une séquence de gène cible et en favorisant l'expression génique en aval de celle-ci chez un humain ou un animal.
